# EUROPÄISCHE PATENTSCHRIFT

(11) **EP 2 169 391 B1**
(45) Veröffentlichungstag und Bekanntmachung des Hinweises auf die Patenterteilung: **03.04.2013**
(21) Anmeldenummer: 08017224.0
(22) Anmeldetag: 30.09.2008
(51) Int. Cl.: G01N 27/07, G01N 33/487, B01L 3/00

(54) **Probenkammerhalter zum Haltern einer Probenkammer und System bestehend aus der Probenkammer und dem Probenkammerhalter**
Device for mounting a sample chamber and system consisting of the sample chamber and the mounting device
Dispositif de fixation d'une chambre d'analyse et système comprenant la chambre d'analyse et le dispositif de fixation

(43) Veröffentlichungstag der Anmeldung: 31.03.2010
(73) Patentinhaber: ibidi GmbH, 82152 Martinsried (DE)
(72) Erfinder: Kahl, Valentin, 80797 München (DE); Zantl, Roman, 85598 Baldham (DE)
(74) Vertreter: Grünecker, Kinkeldey, Stockmair & Schwanhäusser

(56) Entgegenhaltungen:
- WO-A-03/087410
- WO-A2-2006/097751
- DE-A1- 10 229 210
- US-A1- 2005 004 442
- US-A1- 2005 196 857
- US-A1- 2007 237 204
- US-A1- 2008 057 570
- US-B1- 6 544 734

## Beschreibung

Die Erfindung betrifft eine Probenkammerhalterung und ein System umfassend eine Probenkammer und eine Probenkammerhalterung.

Insbesondere inden Bereichen der Zellbiologie und Medizin werden Probenkammem mit integrierten Leiterbahnen und Elektroden zur Untersuchung von Zellen, insbesondere lebender Zellen, verwendet. Dabei wird unter anderem der Wechselstromwiderstand einer mit Zellen belegten Elektrode als Funktion der Zeit gemessen. Solche Probenkammern werden auch als MEA (Multi Electrode Array) Systeme bezeichnet. Auch chemische und pharmakologische Einsatzmöglichkeiten von MEA-Systemen sind denkbar.

Aus der US 5,187,096 ist beispielsweise ein MEA-System bekannt, welches mehrere Aufnahmeschachte zur Aufnahme der zu untersuchenden Zellen umfasst, in denen Elektroden zur Messung der Impedanz von Zellkulturen angeordnet sind. Die Elektroden sind mit Kontaktleisten verbunden, die wiederum mit Leitungen verbunden sind, die aus dem MEA-System hinausführen. Zur Spannungsversorgung oder zur Verbindung der Kontaktleisten mit Messgeräten werden die Leitungen mit externen Gerätschaften verbunden.

Auch aus der US 5,563,067 ist ein MEA-System in Kombination mit einer Halterung bekannt. Dabei umfasst die Vorrichtung eine auf einem Träger angeordnete Einrichtung zur Aufnahme von Zellen, an deren Boden eine planare Elektrode mit einer Vielzahl von Mikroelektroden angeordnet ist. Es führen wiederum Leiter aus der Einrichtung zur Aufnahme von Zellen nach außen. Diese sind auf der Oberfläche des Trägers angeordnet und umfassen mehrere elektrische Kontaktpunkte zum Anschluss einer Spannungsversorgung oder zur Verbindung der Leiter mit Messgeräten.

WO2006/097751 offenbart eine geklammerte Zellwachstumseinheit, wobei ein Temperatursensor in der Einheit elektrisch von aussen kontaktiert werden kann.

WO2003/087410 offenbart einen Biochip, der in zwei Rahmenelemente einklemmbar ist.

US2005/0196857 offenbart einen Glassprobenhalter, der in zwei Rahmenelemente eingeklemmt ist, wobei die Rahmenelemente magnetisch verbindbar sind.

Elektrische Kontaktpunkte dienen in all diesen Systemen zur Herstellung einer stromleitenden Verbindung zu Leitern im MEA-System. Mit den Leitern sind wiederum Messelektroden innerhalb einer Einrichtung zur Aufnahme von Zellen verbunden. An die Kontaktpunkte kann eine externe Spannungsquelle oder ein geeignetes Messgerät, insbesondere über Kontaktelemente auf der Halterung des MEA-Systems, angeschlossen werden.

All den bekannten Systemen ist gemeinsam, dass die Kontaktpunkte über die Einrichtung zur Aufnahme von Zellen des MEA-Systems hinausragen. Insbesondere bei Systemen, bei denen die Leiter und Elektroden sehr dünnschichtig sind oder auf dünnen Trägerplatten aufgebracht sind, kann dies dazu führen, dass die Kontaktpunkte oder die Leiter, welche diese mit den Elektroden im MEA-System verbinden, leicht beschädigt werden können.

Daher ist es Aufgabe der Erfindung, einen Probenkammerhalter zur elektrischen Kontaktierung einer Probenkammer bereitzustellen, sodass die Kontaktelektroden und/oder Leiterbahnen vor Beschädigung geschützt werden.

Diese Aufgabe wird gelöst durch einen Probenkammer halter nach Anspruch 1 und ein system nach Anspruch 6.

Die Probenkammer zur Untersuchung von Proben umfasst eine Bodenplatte und eine damit verbundene Deckplatte, ein Probenreservoir zur Aufnahme einer Flüssigkeit und/oder einer zu untersuchenden Probe, und eine vollständig in der Bodenplatte, in der Deckplatte oder zwischen Bodenplatte und Deckplatte angeordnete Kontaktelektrode, die mit einem in und/oder auf der Probenkammer angeordneten Leiterelement oder Halbleiterelement stromleitend verbunden ist, wobei die Bodenplatte und/oder die Deckplatte derart ausgebildet sind, dass von außen mit der Kontaktelektrode ein elektrischer Kontakt derart herstellbar ist, dass von außen durch die Bodenplatte oder durch die Deckplatte über die Kontaktelektrode eine stromleitende Verbindung mit dem Leiterelement oder mit dem Halbleiterelement herstellbar ist.

Die Probenkammer erlaubt eine sicherere Kontaktierung der Kontaktelektrode. Insbesondere da die Kontaktelektrode nicht über die Probenkammer hinausragt, ist sie vor Beschädigungen geschützt. Die Probenkammer erlaubt es, von außen einen unmittelbaren also mechanischen Kontakt zwischen der Kontaktelektrode und einem externen Kontaktelement herzustellen, sodass über diesen Kontakt Strom geleitet werden kann. Insbesondere kann ein elektrischer Kontakt mit der Kontaktelektrode von außen durch die Bodenplatte oder durch die Deckplatte hindurch hergestellt werden.

Das Probenreservoir der Probenkammer kann durch eine Vertiefung bzw. eine Nut in der Deckplatte und/oder der Bodenplatte gebildet werden. Im Falle einer Nut bzw. Vertiefung in der Deckplatte und/oder der Bodenplatte auf der Innenseite der Probenkammer wird ein Probenreservoir in Form eines Hohlraums gebildet. Dabei kann der Hohlraum vollständig zwischen Deckplatte und Bodenplatte angeordnet sein. Alternativ kann die Probenkammer ein nach außen offenes Reservoir umfassen. Dafür kann die Bodenplatte oder die Deckplatte ein Loch aufweisen. Das Loch kann ein durchgehendes Loch sein. Die Deckplatte kann ein durchgehendes Loch aufweisen und die Bodenplatte eine in Richtung des Lochs weisende Vertiefung bzw. Nut. Alternativ kann die Bodenplatte ein durchgehendes Loch aufweisen und die Deckplatte eine in Richtung des Lochs weisende Vertiefung bzw. Nut.

Die Probenkammer selbst kann in verschiedenen Geometrien und Formen vorliegen. In einer der einfachsten Ausführungen umfasst die Probenkammer eine planare Bodenplatte, die mit einer Deckplatte verbunden ist. Die Deckplatte kann eine Vertiefung bzw. Nut aufweisen, wobei durch die Vertiefung bzw. Nut in der Deckplatte in Verbindung mit der Bodenplatte ein Probenreservoir gebildet wird. Die Form des Probenreservoirs kann durch die Geometrie der Vertiefung in der Deckplatte bestimmt werden. So kann, zum Beispiel, ein Probenreservoir in Form eines kanalförmigen Hohlraums durch eine längliche Nut in der Deckplatte gebildet werden. In das Probenreservoir kann eine zu untersuchende Probe und/oder eine Flüssigkeit eingebracht werden. Die Probenkammer kann für chemische und/oder biologische Untersuchungen an chemischen und/oder biologischen Proben verwendet werden. Als Probe sind, zum Beispiel, lebende Zellen, Proteine, DNA, Viren etc. möglich. Die Probenkammer kann auch mehrere Probenreservoire umfassen. Insbesondere können die mehreren Probenreservoire fluidisch miteinander verbunden sein. Die Bodenplatte und/oder Deckplatte kann als ein Spritzgussteil ausgebildet sein oder aus mehreren Kunststoffteilen zusammengefügt sein. Die mehreren Kunststoffteile können insbesondere in Form und/oder Material unterschiedlich sein.

Das Leiterelement kann insbesondere als Leiterbahn ausgebildet sein.

Die Probenkammer weist ein Loch auf, in dem die Kontaktelektrode angeordnet ist. Dadurch ist die Kontaktelektrode von außen frei zugänglich und kann mit einem externen Kontaktelement, beispielsweise einem Federkontaktstift, direkt kontaktiert werden. Insbesondere kann die Kontaktelektrode an einer Seitenwand des Lochs und/oder am Boden des Lochs angeordnet sein.

Das Loch konn ein Sackloch in der Deckplatte oder in der Bodenplatte sein. Dies ist vorteilhaft, wenn das Leiterelement oder das Halbleiterelement zumindest teilweise in der Bodenplatte oder in der Deckplatte angeordnet ist. Das Loch kann ein Sackloch in der Probenkammer sein. In diesem Fall kann das Sackloch durch ein durchgehendes Loch in der Bodenplatte und durch die Deckplatte gebildet werden. Alternativ kann das Sackloch durch ein durchgehendes Loch in der Deckplatte und durch die Bodenplatte gebildet werden. Die Kontaktelektrode kann auf der Bodenplatte oder auf der Deckplatte derart angeordnet sein, dass sie wenigstens teilweise am Boden des Sackloches angeordnet ist. Dadurch ist die Probenkammer einfach herstellbar. Insbesondere wenn die Kontaktelektrode am Boden des Sacklochs angeordnet ist, ist sie mit einem einfachen Kontaktstift, insbesondere einem Federkontaktstift, kontaktierbar.

Das Loch kann mit einem elektrisch leitenden Material teilweise oder vollständig gefüllt sein. Dadurch ist die Kontaktelektrode vollständig in der Deckplatte oder in der Bodenplatte angeordnet und von außen unmittelbar zugänglich, und ein elektrischer Kontakt kann mit der Kontaktelektrode hergestellt werden.

Insbesondere kann wenigstens eine der Öffnungen des Loches, insbesondere die äußere Öffnung, in einer Ebene liegen, die parallel zur Grundfläche der Probenkammer ist. Die Grundfläche der Probenkammer kann die Außenfläche sein, auf der die Probenkammer im normalen Betrieb aufliegt. Dadurch kann in einfacher Weise von oben oder unten ein elektrischer Kontakt mit der Kontaktelektrode hergestellt werden.

Die Probenkammer kann eine oder mehrere weitere Kontaktelektroden umfassen. Insbesondere kann jede der Kontaktelektroden vollständig in der Bodenplatte, in der Deckplatte oder zwischen Bodenplatte und Deckplatte angeordnet sein, und jeweils mit einem in und/oder auf der Probenkammer angeordneten Leiter- oder Halbleiterelement stromleitend verbunden sein. Dadurch kann mit mehreren auf und/oder in der Probenkammer angeordneten Leiter-und/oder Halbleiterelementen von außen durch die Bodenplatte oder durch die Deckplatte jeweils über eine Kontaktelektrode eine stromleitende Verbindung hergestellt werden.

Insbesondere kann die Probenkammer eine Mehrzahl von Löchern aufweisen, in denen jeweils eine Kontaktelektrode angeordnet ist. Alternativ können auch mehrere Kontaktelektroden in einem Loch angeordnet sein.

Jedes der Löcher kann jedes der obengenannten Merkmale aufweisen. Jedes der Löcher kann einen Öffnungsquerschnitt aufweisen, der kleiner, größer oder gleich groß der Fläche der jeweils darin angeordneten Kontaktelektrode ist. Wenn mehrere Kontaktelektroden in einem Loch angeordnet sind, kann der Öffnungsquerschnitt vorzugsweise größer als die Summe der Flächen der Kontaktelektroden sein, um diese voneinander elektrisch zu isolieren.

Die Probenkammer weist eine Messelektrode und/oder eine Anregungselektrode auf, welche jeweils mit einer Kontaktelektrode über jeweils ein Leiter- oder Halbleiterelement stromleitend verbunden sind. Eine Messelektrode und/oder Anregungselektrode kann insbesondere in einem Probenreservoir, beispielsweise in Form eines Hohlraums, angeordnet sein. Messelektroden können beispielsweise der Messung der Impedanz einer Zellkultur im Probenreservoir dienen. Anregungselektroden können zur Anlegung einer Spannung an die Probe dienen.

Insbesondere kann die Kontaktelektrode, die Messelektrode, die Anregungselektrode und/oder das Leiterelement Gold, Platin, Titan oder TiNi (Titan-Nickel) umfassen. In einer Ausführungsform kann das Halbleiterelement ITO (Indiumzinnoxid) umfassen.

Das Halbleiterelement kann insbesondere der Temperierung der Probenkammerdienen.

Das Leiterelement und die Kontaktelektrode und/oder die Messelektrode bzw. die Anregungselektrode können aus einem Material gefertigt sein. Dadurch wird die Fertigung der Probenkammer vereinfacht und die elektrischen Eigenschaften von Leiterelement und Kontaktelektrode, Messelektrode und/oder Anregungselektrode sind einheitlich. Insbesondere können das Leiterelement und die Kontaktelektrode aus einem Material bestehen.

In einer Weiterbildung können das Leiterelement, das Halbleiterelement, die Kontaktelektrode, die Messelektrode und/oder die Anregungselektrode planar sein. Insbesondere bei der Kontaktelektrode kann dies die Herstellung eines stabilen elektrischen Kontakts von außen erleichtern.

Das Leiterelement, das Halbleiterelement, die Messelektrode und/oder die Anregungselektrode können 10 nm bis 5 µm, insbesondere 10 nm bis 300 nm, dick sein.

In einer Weiterbildung können die Kontaktelektrode, die Messelektrode, die Anregungselektrode, das Halbleiterelement und/oder das Leiterelement auf der Bodenplatte und/oder der Deckplatte angeordnet sein, wobei die Kontaktelektrode vollständig zwischen Bodenplatte und Deckplatte angeordnet ist. Dies hat den Vorteil, dass die Herstellung der Probenkammer vereinfacht wird.

Insbesondere können das Leiterelement und/oder das Halbleiterelement vollständig zwischen der Bodenplatte und der Deckplatte oder vollständig auf der Außenseite der Probenkammer oder teilweise zwischen der Bodenplatte und der Deckplatte und teilweise auf der Außenseite der Probenkammer angeordnet sein. Wenn sich das Leiterelement und/oder das Halbleiterelement vollständig zwischen der Bodenplatte und der Deckplatte befinden hat dies den Vorteil, dass sie gegen Beschädigungen geschützt sind.

Das Leiterelement und/oder das Halbleiterelement können vollständig oder teilweise innerhalb der Bodenplatte und/oder Deckplatte der Probenkammer angeordnet sein. Insbesondere können die Messelektrode und/oder die Anregungselektrode teilweise innerhalb der Bodenplatte und/oder Deckplatte der Probenkammer angeordnet sein. Dies ist von Vorteil, wenn nur ein Teil der Fläche der Messelektrode und/oder Anregungselektrode mit der Probe in Kontakt kommen soll.

Das Leiterelement, das Halbleiterelement, die Messelektrode und/oder die Anregungselektrode können vollständig oder teilweise in einem Probenreservoir angeordnet und insbesondere vollständig oder teilweise gegen das Probenreservoir elektrisch isoliert sein. Insbesondere können die Messelektrode und/oder die Anregungselektrode durch Teile des Leiterelements oder des Halbleiterelements gebildet werden, die nicht oder teilweise gegen das Probenreservoir isoliert sind. Dadurch ist die Probenkammer einfach und effizient herstellbar. Die Größe der Elektrode kann durch die Größe der isolierenden Schicht bestimmt werden.

In einer Ausführungsform kann die Probenkammer ein, insbesondere mit der Kontaktelektrode stromleitend verbundenes, Heizelement umfassen. Das Heizelement kann Teil der Probenkammer, also in die Probenkammer integriert sein. Insbesondere kann das integrierte Heizelement auf und/oder in der Bodenplatte und/oder Deckplatte angeordnet sein. Insbesondere kann das Heizelement das Halbleiterelement umfassen. Beispielsweise kann die Außenseite der Probekammer vollständig oder teilweise das Halbleiterelement umfassen. Insbesondere kann auf und/oder in der Bodenplatte das Halbleiterelement, insbesondere ITO, angeordnet sein. Dadurch kann eine flächige Temperierung der gesamten Bodenplatte erfolgen. Spezielle temperaturabhängige Widerstandselemente, beispielsweise PT100, können verwendet werden, um die Temperatur zu bestimmen und über einen Regelkreislauf rückgekoppelt zu steuern.

In einer Weiterbildung können die Deckplatte und die Bodenplatte unmittelbar, insbesondere flächig, miteinander verbunden sein. Dadurch können sich Bodenplatte und Deckplatte gegenseitig stabilisieren und damit auch eine, insbesondere zwischen Bodenplatte und Deckplatte angeordnete Kontaktelektrode vor Beschädigungen schützen. Insbesondere wenn die Bodenplatte oder die Deckplatte dünn ist, beispielsweise zwischen 1 µm und 300 µm dick, ist dies von Vorteil. Des weiteren ist die Probenkammer dadurch leichter und kompakter herstellbar. Insbesondere können Deckplatte und Bodenplatte fluchtend miteinander verbunden sein.

Insbesondere können die Deckplatte und die Bodenplatte zerstörungsfrei lösbar oder nicht zerstörungsfrei lösbar miteinander verbunden sein. Beispielsweise können Deckplatte und Bodenplatte verklebt oder verschweißt, insbesondere laserverschweißt, ultraschallverschweißt, thermoverschweißt oder lösungsmittelverschweißt, sein.

Die Deckplatte und die Bodenplatte können zum Beispiel durch Verkleben mit PDMS zerstörungsfrei lösbar verbunden sein. Insbesondere wenn das Leiterelement auf der Bodenplatte und der Deckplatte angeordnet ist, ist es vorteilhaft, wenn Deckplatte und Bodenplatte verschweißt sind. Bodenplatte und Deckplatte können flüssigkeitsdicht verbunden sein. Insbesondere Lösungsmittelverschweißen und Thermoverschweißen haben dabei den Vorteil, dass bei Verwendung von nicht isolierten Leiterelementen, die zwischen Bodenplatte und Deckplatte angeordnet sein können, keine Flüssigkeit beispielsweise entlang der Leiterelemente diffundiert.

Die Bodenplatte und/oder die Deckplatte können Glas, insbesondere Deckglas, oder Kunststoff, insbesondere COC (Cyclo-olefin Copolymer), COP (Cyclo-olefin Polymer), PC (Polycarbonat), PS (Polystyrol), PE (Polyethylen) oder PMMA (Polymethylmethacrylat), umfassen.

Die Bodenplatte und/oder Deckplatte können eine vorherbestimmte Eigenfluoreszenz, die insbesondere kleiner oder gleich der Eigenfluoreszenz von COC oder COP oder eines herkömmlichen Deckglases ist, und/oder einen vorherbestimmten Brechungsindex, insbesondere > 1,2 und/oder < 1,7, aufweisen.

Insbesondere kann die Eigenfluoreszenz kleiner oder gleich der Eigenfluoreszenz eines herkömmlichen Deckglases (beispielsweise reinweißes Glas der hydrolytischen Klasse 1 (wie Menzel-Deckglas, insbesondere mit der Stärke Nr. 1,5) sein. Der vorherbestimmte Brechungsindex kann insbesondere > 1,2 und/oder < 1,7, sein. Mit einem derart optisch hochwertigen Material lassen sich in vorteilhafter Weise Mikroskopieuntersuchungen durchführen. Beispielsweise kann die Doppelbrechung so gering sein, dass DIC (Differential Interference Contrast) möglich ist. Eine geringe Eigenfluoreszenz erlaubt die Durchführung von Fluoreszenzmessungen.

Insbesondere kann die Bodenplatte und/oder Deckplatte in einem für Mikroskopie verwendeten Frequenzbereich der elektromagnetischen Strahlung entspiegelt sein. Dadurch kann die Transmission durch die Bodenplatte und/oder Deckplatte erhöht werden, sodass Einzelmolekülmessungen mit Hilfe von Fluoreszenz möglich sind. Für die Entspiegelung kann die Bodenplatte und/oder die Deckplatte eine Beschichtung aufweisen. Beispielsweise kann eine ITO-Schicht auf der Bodenplatte und/oder Deckplatte angeordnet sein. Die Dicke der ITO-Schicht kann so gewählt werden, dass die Bodenplatte und/oder Deckplatte in einem für Mikroskopie verwendeten Frequenzbereich der elektromagnetischen Strahlung entspiegelt sind. Beispielsweise kann die Dicke der ITO-Schicht λ/2 bis 4λ betragen, um eine Entspiegelung zu erreichen, wobei λ die verwendete Wellenlänge angibt. Insbesondere kann die Wellenlänge λ im Bereich von 300 nm bis 700 nm liegen. In diesem Fall kann die Dicke der Beschichtung zwischen 200 nm und 1 µm liegen. Insbesondere kann die ITO-Schicht auch zur Temperierung der Probenkammer verwendet werden.

Die Bodenplatte kann wenigstens teilweise planar sein. Der planare Teil der Bodenplatte kann zwischen 1µm und 300 µm, insbesondere zwischen 60µm und 220 µm, dick sein. Dies ist von Vorteil, um hochauflösende Mikroskopie durch die Bodenplatte zu ermöglichen, da in diesem Fall der Arbeitsabstand des Objektivs zur Probe klein sein kann. Beispielsweise kann dann mit hochauflösenden Objektiven, beispielsweise mit 63x oder 100x bzw. mit Aperturen von 1,2 und größer, gearbeitet werden.

In einer Ausführung kann die Deckplatte wenigstens teilweise planar sein, und der planare Teil der Deckplatte kann mindestens 0.5 mm, insbesondere zwischen 1 mm und 2 mm, dick sein. Dadurch kann die mit der Deckplatte verbundene Bodenplatte stabilisiert werden, insbesondere wenn die Bodenplatte sehr dünn, also beispielsweise zwischen 1 µm und 300 µm dick ist.

Die Bodenplatte und/oder die Deckplatte können eine Öffnung umfassen, um das Probenreservoir, insbesondere in Form eines Hohlraums, mit einer Flüssigkeit und/oder einer zu untersuchenden Probe zu befüllen, und/oder eine Flüssigkeit und/oder eine zu untersuchende Probe zu entnehmen.

Die Probenkammer kann ein oder mehrere weitere, insbesondere nach außen offene, Reservoire umfassen. Diese weiteren Reservoire können fluidisch jeweils mit einem oder mehreren Probenreservoire verbunden sein. Dadurch ist ein Hinzufügen oder Entnehmen einer Flüssigkeit und/oder einer Probe in bzw. aus dem Probenreservoir möglich. Insbesondere können zwei nach außen offene Reservoire auf zwei, insbesondere gegenüberliegenden Seiten, eines Probenreservoirs, insbesondere in Form eines kanalförmigen Hohlraums, angeordnet sein. Dadurch ist ein rascher Austausch der im Probenreservoir befindlichen Flüssigkeit und/oder Probe möglich.

Insbesondere können die Bodenplatte und/oder Deckplatte ein Verbindungselement, insbesondere zur Verbindung der Probenkammer mit Vorrichtungen zum Absaugen und/oder Hinzufügen einer Flüssigkeit und/oder einer Probe aus dem bzw. in ein Probenreservoir und/oder weiteres Reservoir der Probenkammer, umfassen. Das Verbindungselement kann insbesondere die Form eines Lueradapters haben. Das Verbindungselement der Bodenplatte und/oder Deckplatte kann die Öffnung umfassen. Das Verbindungselement kann insbesondere fluidisch mit dem Probenreservoir und/oder einem weiteren Reservoir verbunden sein. Dadurch kann eine konische Steckverbindung, beispielsweise mit einer Pipettenspitze, zum Befüllen oder Leeren des Reservoirs oder zum luft- und flüssigkeitsdichten Verschließen, insbesondere mit einem konischen Stecker oder anderen Abdichtelementen, gebildet werden. Dazu kann die Öffnung und das Verbindungselement direkt über dem Probenreservoir, insbesondere in Form eines Hohlraums, oder über einem weiteren Reservoir angeordnet sein. Das Verbindungselement kann auch über einen Kanal mit dem Probenreservoir und/oder einem weiteren Reservoir verbunden sein. Die Vorrichtung zum Absaugen und/oder Hinzufügen einer Flüssigkeit und/oder einer Probe aus dem bzw. in das Probenreservoir oder ein weiteres Reservoir der Probenkammer kann beispielsweise eine Pipette oder ein Schlauch sein. Das Verbindungselement kann konisch geformt sein. Dadurch kann eine dichte Verbindung hergestellt werden.

In einer Weiterbildung kann die Probenkammer derart dimensioniert sein, dass das Volumen des Probenreservoirs oder eines weiteren Reservoirs in dem Bereich von 5 µl bis 5000 µl, insbesondere zwischen 100 µl und 500 µl, liegt. Damit ist die Probenkammer für Mikrofluiduntersuchungen verwendbar. Dies ist von Vorteil, wenn mit kleinen Mengen biologischer oder chemischer Proben gearbeitet wird, oder wenn das mikrofluide Verhalten der Probe untersucht werden soll.

Die Erfindung stellt einen Probenkammerhalter zum Haltern einer Probenkammer bereit, gemass Anspruch 1

Insbesondere kann der Probenkammerhalter eine der zuvor beschriebenen Probenkammern haltern.

Durch diesen Probenkammerhalter wird eine sichere Kontaktierung einer Probenkammer möglich. Ein weiterer Vorteil dieses Probenkammerhalters ist, dass er aufgrund der Verbindungsvorrichtung, die wenigstens ein magnetisches und/oder magnetisierbares Element umfasst, kompakter und kleiner sein kann als ein Probenkammerhalter, dessen Verbindungselement beispielsweise schraubbare Elemente umfasst.

Insbesondere kann die Verbindungseinrichtung wenigstens einen oder mehrere Dauermagnete umfassen. Dadurch ist kein Verschrauben der beiden Rahmenelemente der Probenkammer notwendig und der Probenkammerhalter kann so kompakt und miniaturisiert ausgeführt sein, dass dieser in eine Inkubationsvorrichtung integriert werden kann. Beispielsweise können vier Dauermagnete an jedem Rahmenelement verwendet werden.

In einer Weiterbildung können das erste Rahmenelement und/oder das zweite Rahmenelement Vertiefungen zum Aufnehmen der Probenkammer aufweisen. Damit wird ein sicheres Positionieren und Fixieren der Probenkammer erleichtert. Insbesondere kann dadurch die korrekte Anordnung der Probenkammer für den Nutzer erleichtert werden.

Das elektrische Kontaktelement des Probenkammerhalters kann einen Federkontakt umfassen. Insbesondere kann das elektrische Kontaktelement des Probenkammerhalters einen Federkontaktstift umfassen. Dadurch kann beispielsweise mit einer oben beschriebenen Probenkammer ein stabiler elektrischer Kontakt hergestellt werden.

In einer Weiterbildung können das erste Rahmenelement und/oder das zweite Rahmenelement derart ausgebildet sein, dass eine Probe in der Probenkammer mikroskopierbar ist. Dies ist von Vorteil, wenn nicht nur die elektrischen Leitungseigenschaften der Probe in der Probenkammer untersucht werden sollen, sondern auch Mikroskopieuntersuchungen stattfinden sollen.

Insbesondere können das erste Rahmenelement und/oder das zweite Rahmenelement ein Durchgangsloch oder einen transparenten Bereich, insbesondere aus Glas oder Kunststoff, umfassen. Dadurch kann ein Mikroskopobjektiv an die Probe in der Probenkammer herangeführt werden. Der beispielhafte transparente Bereich des ersten und/oder zweiten Rahmenelements ist dabei wenigstens für jenen Frequenzbereich des elektromagnetischen Spektrums transparent, in dem die Mikroskopieuntersuchung durchgeführt werden soll.

Der transparente Bereich im ersten Rahmenelement und/oder im zweiten Rahmenelement kann ein Material umfassen, das optische Eigenschaften besitzt, wie sie oben beispielhaft für die Bodenplatte und/oder die Deckplatte der Probenkammer beschrieben wurden.

In einer Weiterbildung kann der Probenkammerhalter, insbesondere durch eine ITO-Beschichtung, temperierbar sein. Der Probenkammerhalter kann passiv temperierbar sein, indem er mit einem temperierbaren Element lösbar verbunden wird. Insbesondere kann der Probenkammerhalter selbst ein Temperierelement umfassen. Beispielsweise kann der Probenkammemhalter vollständig oder teilweise, insbesondere in einem transparenten Teil des ersten und/oder zweiten Rahmenelements, mit ITO beschichtet sein. Dadurch kann eine flächige Temperierung erfolgen. Spezielle temperaturabhängige Widerstandselemente, beispielsweise PT100, können verwendet werden, um die Temperatur zu bestimmen und über einen Regelkreislauf rückgekoppelt zu steuern.

Des weiteren kann der Probenkammerhalter eine Verbindungseinrichtung zur elektrischen Kontaktierung des Probenkammerhalters von außen umfassen. Dadurch kann die Probenkammer über den Probenkammerhalter an ein externes Gerät, insbesondere eine Spannungsquelle oder ein Messgerät, angeschlossen werden. Dafür wird der Probenkammerhalter über die Verbindungseinrichtung kontaktiert. Wenigstens ein Kontaktelement des Probenkammerhalters stellt daraufhin einen elektrischen Kontakt zur Kontaktelektrode der Probenkammer her. Die Verbindungseinrichtung kann ein Steckverbindungselement oder ein Kabel umfassen. Der Probenkammerhalter kann mit externen Geräten, beispielsweise mit einer Spannungsquelle oder mit einem Messgerät, verbunden sein. Diese Verbindung kann über ein elektrisch leitende Kabelverbindung erfolgen, wobei das Kabel mit dem mindestens einem elektrischen Kontaktelement des Probenkammerhalters elektrisch leitend verbunden ist. Alternativ kann auch ein Steckverbindungselement derart ausgebildet sein, dass eine leitende Verbindung zu einem externen Element, beispielsweise einer Basisplatte hergestellt werden kann, wobei dieses externe Element wiederum entsprechende leitende Verbindungen umfassen kann.

Die Erfindung stellt weiterhin ein System, umfassend eine Probenkammer wie oben beschrieben und einen wie oben beschriebenen Probenkammernhalter, bereit.

Durch solch ein System wird eine sichere Kontaktierung einer erfindungsgemäßen Probenkammer möglich, wobei die Kontaktelektroden vor Beschädigung geschützt werden.

Weitere Merkmale und Vorteile werden nachfolgend an Hand der beispielhaften Figuren erläutert.
- Figur 1: zeigt eine Drahtgitterzeichnung einer beispielhaften Probenkammer;
- Figur 2: zeigt eine Explosionszeichnung der beispielhaften Probenkammer aus Figur 1;
- Figur 3: zeigt eine perspektivische Ansicht eines beispielhaften Systems umfassend eine Probenkammer und einen Probenkammerhalter;
- Figur 4: zeigt eine Drahtgitterzeichnung der Anordnung aus Figur 3;
- Figur 5: zeigt eine perspektivische Ansicht des zusammengesetzten Systems aus Figur 3;

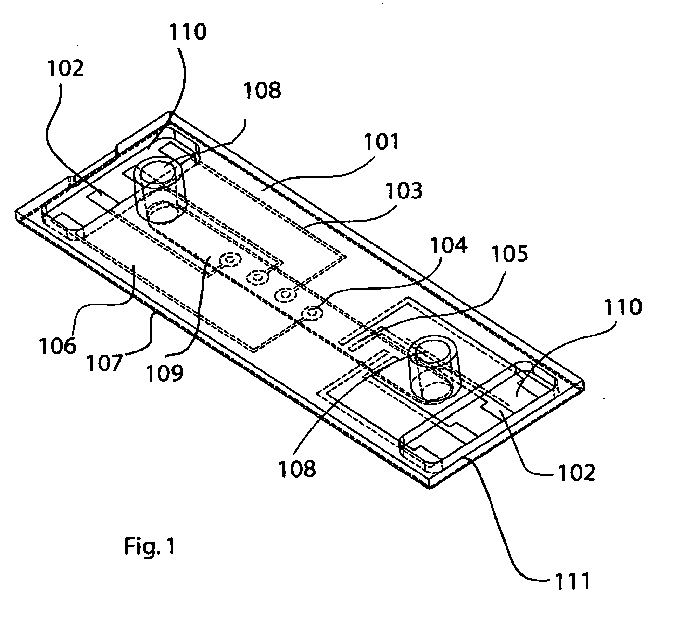

Figur 1 zeigt ein Beispiel einer Probenkammer 101 mit Kontaktelektroden 102, Leiterelementen und Anregungs- und Messelektroden 104, 105. In diesem Ausführungsbeispiel sind die Leiterelemente als Leiterbahnen 103 ausgebildet. Die Probenkammer ist in diesem Ausführungsbeispiel rechteckig. Der planare Teil der Probenkammer kann beispielsweise 75 mm x 25 mm x 2 mm groß sein. Jeweils vier Kontaktelektroden 102 befinden sich am Boden von jeweils einem der zwei Sacklöcher 110. Die zwei Sacklöcher 110 sind dabei an den gegenüberliegenden Enden der Längsachse der Probenkammer 101 angeordnet. Jeweils eine Kontaktelektrode 102 ist über jeweils eine Leiterbahn 103 mit jeweils einer Messelektrode 104 bzw. Anregungselektrode 105 verbunden. Die Anregungselektroden 105 bzw. Messelektroden 104 sind in einem als kanalförmigen Hohlraum 109 ausgebildeten Probenreservoir angeordnet. Teile der Deckplatte 106 sind als nach außen offenes weiteres Reservoir 108 ausgebildet. Die beiden weiteren nach außen offenen Reservoire 108 sind an zwei gegenüberliegenden Seiten des Probenreservoirs in Form eines kanalförmigen Hohlraums 109 angeordnet. Die Deckplatte 106 kann als ein Spritzgussteil ausgebildet sein oder aus mehreren Kunststoffteilen zusammengefügt sein.

Die Kontaktelektroden 102 könnten auch jeweils in einem Loch angeordnet sein. Dabei könnte jedes Loch ein durchgehendes Loch oder ein Sackloch sein. Insbesondere können die Kontaktelektroden am Boden und/oder an einer Seitenwand des Lochs angeordnet sein. Die Kontaktelektroden 102 können in der Bodenplatte 107 oder der Deckplatte 106, insbesondere vollständig oder teilweise im Loch, angeordnet sein. Dadurch ist die Kontaktelektrode von außen unmittelbar zugänglich und ein elektrischer Kontakt kann mit der Kontaktelektrode hergestellt werden. Insbesondere wenn jeweils eine Kontaktelektrode 102 in einem Loch angeordnet ist, kann das Loch auch vollständig mit einem leitenden Material gefüllt sein.

Durch die Anregungselektroden 105 können zum Beispiel definierte Ströme oder Spannungen mit vorherbestimmten Frequenzen angelegt werden. Auch ist es möglich, die Probenkammer 101 durch definierten Stromfluss durch die Elektroden zu temperieren. Insbesondere können entsprechende Anordnungen der Leiterbahnen 103 oder Halbleiterelemente zur Temperierung der Probenkammer 101 verwendet werden. Insbesondere kann für diese Anwendung ITO eingesetzt werden.

Durch die Messelektroden 104 können, je nach Ausführung, elektrische Signale zur Bestimmung von Spannungen, Strömen oder auch Temperaturen aufgenommen werden.

Die Probenkammer 101 kann im Wesentlichen aus einer Deckplatte 106 und einer Bodenplatte 107 bestehen. Dabei kann die Deckplatte 106 eine Vertiefung bzw. Nut oder ein Loch aufweisen. Die Bodenplatte 107 kann aus einer planaren Fläche, beispielsweise einem Glasträger oder einer Kunststofffolie, bestehen. Durch die Vertiefung bzw. Nut auf der Innenseite der Probenkammer wird ein Probenreservoir in Form eines kanalförmigen Hohlraums 109 gebildet.

In diesem Ausführungsbeispiel befinden sich die Anregungselektroden 105 und Messelektroden 104 am Boden des Probenreservoirs in Form eines kanalförmigen Hohlraums 109, der über die weiteren nach außen offenen Reservoire 108 befüllbar ist, die mit diesem fluidisch verbunden sind. Die Anregungs- und Messelektroden 105, 104 können auch auf dem Boden eines nach außen offenen Probenreservoirs angeordnet sein.

In dem hier gezeigten Beispiel sind Kontaktelektroden 102, Messelektroden 104, Anregungselektroden 105 und Leiterbahnen 103 auf der Bodenplatte 107 angeordnet. Dabei befinden sich die Kontaktelektroden 102 am Boden eines Sacklochs 110. Werden die Bodenplatte 107 und die Deckplatte 106, insbesondere flächig, miteinander verbunden, sind die Kontaktelektroden 102 fixiert, da die Deckplatte 106, beispielsweise in Form eines "Stegs" 111, für eine entsprechende Stabilität sorgt. Würde die Bodenplatte 107 mit den Kontaktelektroden 102 über den Rand der Deckplatte 106 hinausragen, würde dieser Teil der Bodenplatte 107 leicht abbrechen, insbesondere wenn es sich um ein normales Deckglas (170 µm dick) oder um eine Kunststofffolie (100 bis 200 µm dick) handelt. Auch könnten durch entsprechende Biegung gegenüber der Deckplatte 106 Risse in den Leiterbahnen 103 entstehen.

Die Messelektroden 104 und Anregungselektroden 105 befinden sich hier auf der Bodenplatte 107. Es sind jedoch auch Anordnungen denkbar, in denen sich die Messelektroden 104 und/oder Anregungselektroden 105 an der Deckplatte 106 oder an Bodenplatte 107 und Deckplatte 106 befinden. Durch das Verbinden der Bodenplatte 107 und der Deckplatte 106 kann in letzterem Fall ein elektrischer Kontakt zwischen den beispielsweise auf der Bodenplatte 107 angeordneten Kontaktelektroden 102 und den beispielsweise an der Deckplatte 106 angebrachten Messelektroden 104 realisiert werden. Dabei sind Verbindungstechnologien ohne die Verwendung von Klebern (zum Beispiel Verschweißtechnologien) vorteilhaft.

Bei den Elektroden und/oder Leiterbahnen 103 kann es sich insbesondere um aufgedampfte oder aufgedruckte leitende Schichten aus Gold, Platin, Titan etc. handeln, die Schichtdicken von 10 nm bis 5 µm aufweisen. Durch das Verbinden der Deckplatte 106 und der Bodenplatte 107 sind Teile der Leiterbahn 103, die sich im Verbindungsbereich der Bodenplatte 107 und der Deckplatte 106 befinden, vollständig isoliert. Möchte man nur kleine Elektroden als Messelektroden 104 verwenden, können die entsprechenden Messelektroden 104 in einem nach außen offenen Probenreservoir und/oder in einem Probenreservoir in Form eines kanalförmigen Hohlraums 109 mit einer lsolationsschicht überzogen sein. Diese kann sich nur über den relevanten Bereich des Probenreservoirs erstrecken oder über die gesamte Leiterbahn 103. Die Verbindung der Deckplatte 106 mit der Bodenplatte 107 kann durch Verkleben oder Verschweißen erfolgen. Insbesondere bei nichtisolierten Leiterbahnen 103 hat sich das Thermoverschweißen oder das Lösungsmittelverschweißen bewährt.

Die Probenkammer kann beispielsweise Merkmale wie in DE 101 48 210 dargestellt aufweisen.

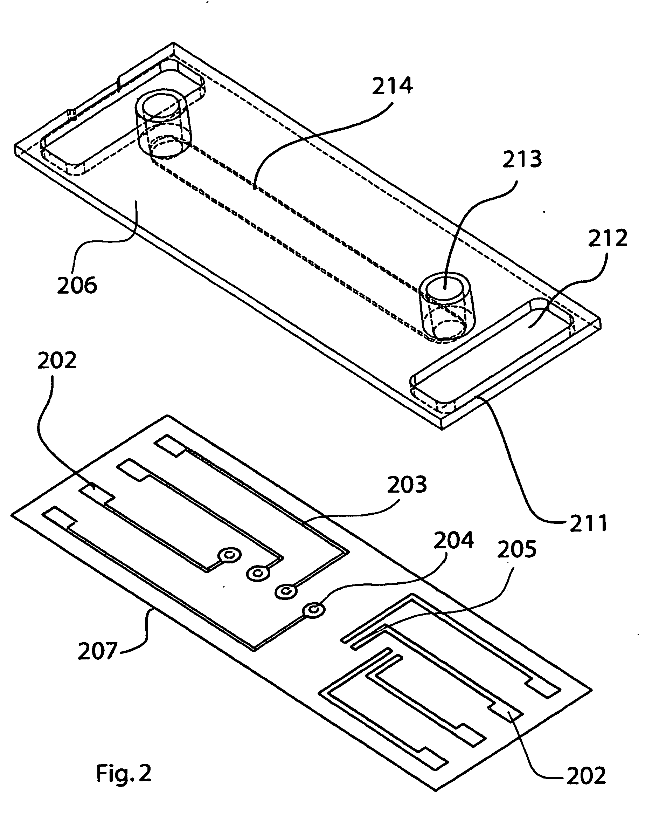

Figur 2 zeigt eine Explosionszeichnung der beispielhaften Probenkammer aus Figur 1. In der Deckplatte 206 sind Löcher 212 und 213 angeordnet. Auf der Bodenplatte 207 sind Leiterbahnen 203 angeordnet. Diese können als nichtisolierte oder teilweise isolierte Leiterbahnen 203 ausgeführt sein. Jeweils eine Kontaktelektrode ist mit jeweils einer Messelektrode 204 bzw. Anregungselektrode 205 über jeweils eine Leiterbahn 203 elektrisch leitend verbunden. In der Deckplatte 206 ist eine Nut 214 angeordnet, durch die, in Verbindung mit der Bodenplatte 207, in der Probenkammer ein zwischen Deckplatte 206 und Bodenplatte 207 angeordnetes Probenreservoir in Form eines Hohlraums gebildet wird. In der Deckplatte 206 ist ein durchgehendes Loch 213 derart angeordnet, dass es in der Probenkammer die Öffnung eines weiteren nach außen offenen Reservoirs bildet.

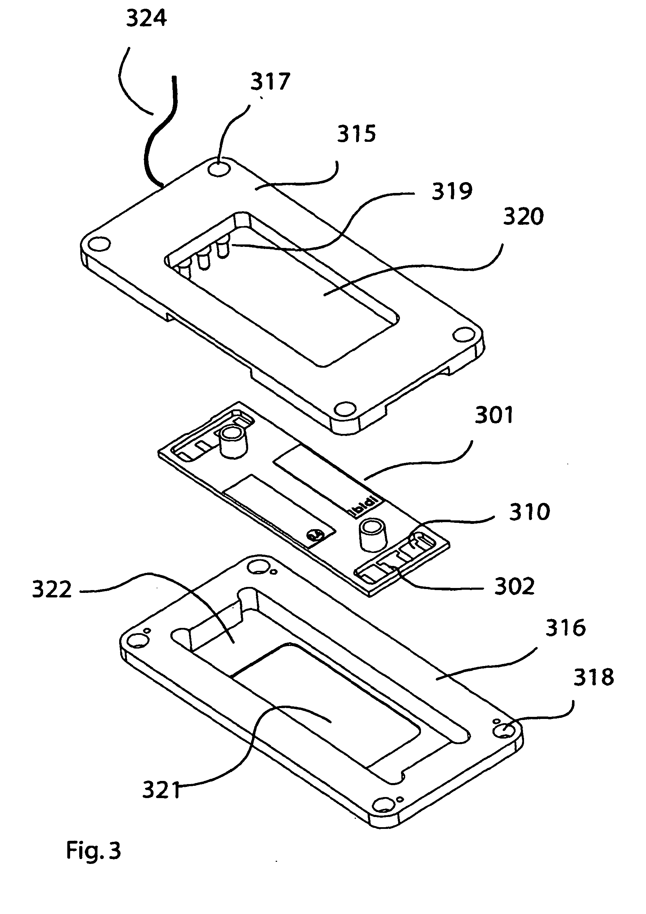

Figur 3 zeigt eine perspektivische Ansicht eines beispielhaften Systems umfassend eine Probenkammer 301 und einen Probenkammerhalter. Der Probenkammerhalter umfasst ein erstes Rahmenelement 315 und ein zweites Rahmenelement 316. In dieser Ausführung werden die Rahmenelemente durch die Kraft der eingebrachten Magnete 317, 318 zusammengehalten, wenn das erste Rahmenelement 315 und das zweite Rahmenelement 316 aufeinandergelegt werden. Jeweils vier Magnete sind im ersten Rahmenelement 315 und im zweiten Rahmenelement 316 angeordnet. Jeweils ein Magnet ist pro Ecke der rechteckigen Rahmenelemente angeordnet. Dabei müssen die Magnete so stark sein, dass ein Kontaktelement 319, insbesondere ein Federkontakt, fest auf die Kontaktelektrode 302 der Probenkammer 301 gepresst wird. Es können zylinderförmige Dauermagnete 317, 318 verwendet werden, mit einem Durchmesser von 5 mm und einer Höhe von 4 mm, die insbesondere so angeordnet werden können, dass die Magnete im zusammengesetzten Probenkammerhalter einen Abstand von höchstens 2 mm haben. Die Magnete können dabei so stark sein, dass eine 2 mm dicke, im Probenkammehalter fixierte, planare Platte aus COC mit einer leichten Krümmung von 50 µm auf 75 mm gerade gepresst wird.

Dadurch reicht die Kraft aus, um das Kontaktelement 319 des Probenkammerhalters, insbesondere einen Federkontakt, fest mit der Kontaktelektrode 302 der Probenkammer zu verbinden. Dadurch ist kein Verschrauben der beiden Rahmenelemente des Probenkammerhalters notwendig und der Probenkammerhalter kann so kompakt und miniaturisiert ausgeführt sein, dass dieser in eine Inkubationsvorrichtung integriert werden kann.

In der gezeigten Ausführung sind mehrere Kontaktelemente 319, insbesondere Kontaktierungsstifte oder Federkontakte, in das erste Rahmenelement 315 eingebracht. Im zweiten Rahmenelement 316 des Probenkammerhalters ist eine entsprechende Vertiefung 322 zum Einlegen der Probenkammer 301 vorgesehen. Die Bereiche 320 und 321 sind vorzugsweise offen. Somit kann ungehindert die Probe in der Probenkammer 301 betrachtet werden. Die Bereiche 320 oder 321 können jedoch auch abgedeckt sein. So könnte der Bereich 321 und/oder 320 zum Beispiel mit transparentem Glas oder Kunststoff abgedeckt sein. Das Glas oder der Kunststoff können insbesondere temperierbar sein (zum Beispiel durch eine ITO-Beschichtung). Der gesamte Probenkammerhalter kann wiederum selber temperierbar sein. Durch ein Kabel 324 können alle elektrischen Elemente und/oder Kontakte im Probenkammerhalter angesteuert werden.

Zur passiven Temperierung des gesamten Probenkammerhalters kann der Probenkammerhalter in eine entsprechend temperierbare Platte eingelegt werden. Diese Platte kann so ausgeführt sein, dass sie wiederum in den üblichen Mikroskophalter für eine 96-Well Platte passt. Anstelle des Kabels 324, das aus dem Probenkammerhalter in die entsprechenden (elektrischen/elektronischen) Gerätschaften führt, kann auch eine direkte Steckverbindung zwischen dem Probenkammerhalter und der Platte vorhanden sein. In diesem Fall könnte das Verbindungskabel wiederum direkt mit der Platte verbunden sein, in die entsprechende Verbindungen eingebracht sind.

In der Probenkammer 301 ist ein Sackloch 310 angeordnet, in dem sich die Kontaktelektroden 302 befinden. Befindet sich die Probenkammer 301 im zusammengefügten Probenkammerhalter, drücken die Kontaktelemente, insbesondere Federkontakte 319, auf die Kontaktelektroden 302. Eine feste Kontaktierung wird durch die im ersten und zweiten Rahmenelement angeordneten Magnete 317, 318 erreicht, sodass im zusammengesetzten Probenkammerhalter die Kontaktelemente 319 auf die Kontaktelektroden 302 gedrückt werden.

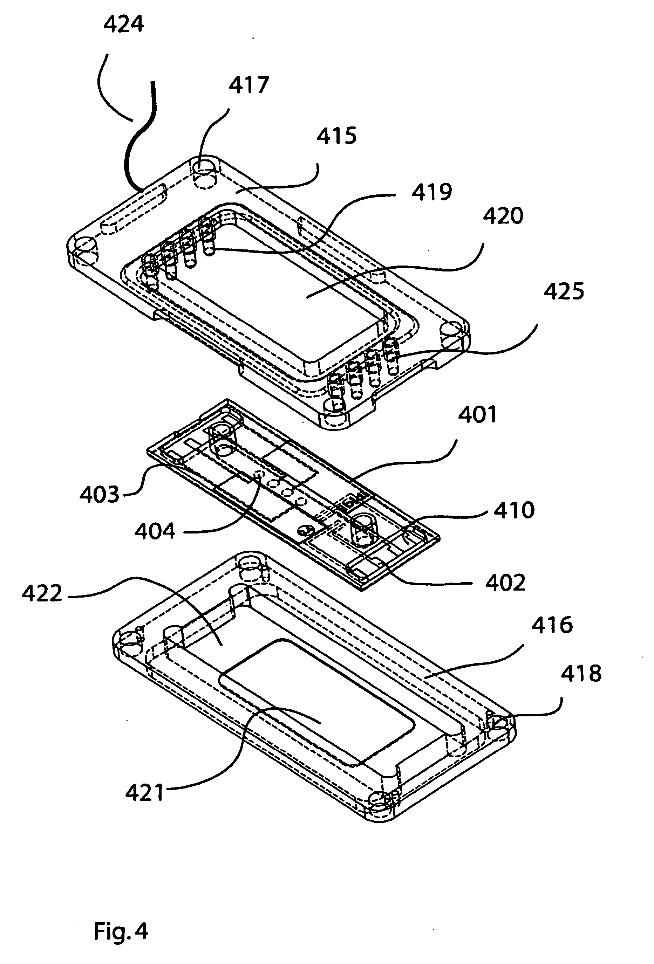

Figur 4 zeigt das beispielhafte System umfassend eine Probenkammer 401 und einen Probenkammerhalter aus Figur 3 in einer Drahtgitterzeichnung. Somit werden neben allen in Figur 3 gezeigten Merkmalen auch weitere Kontaktelemente 425 des Probenkammerhalters erkennbar. Ebenfalls sichtbar werden neben den Kontaktelektroden 402 die zu den Anregungs- oder Messelektroden 404 führenden Leiterelemente, die als Leiterbahnen 403 ausgebildet sind. Durch ein Kabel 424, hier nur schematisch angedeutet, können alle elektrischen Elemente und/oder Kontakte im Probenkammerhalter kontaktiert werden.

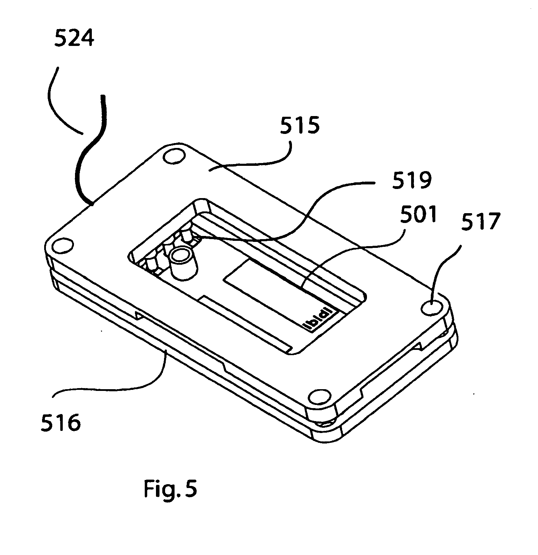

Figur 5 zeigt eine perspektivische Ansicht des zusammengesetzten beispielhaften Systems aus Figur 3. Dabei werden die Rahmenelemente 515, 516 durch die Magnete 517 in einer vorherbestimmten relativen Position zueinander gehalten. Die Kontaktelemente 519 des Probenkammerhalters sind direkt mit den Kontaktelektroden der fixierten Probenkammer 501 verbunden. Durch einen offenen Bereich im ersten Rahmenelement 515 ist die fixierte Probekammer 501 von außen zugänglich. Insbesondere die Öffnung 513 in der Deckplatte der Probenkammer 501 ist zugänglich, wodurch das weitere nach außen offene Reservoir der Probenkammer 501 befüllt bzw. entleert werden kann.

Eine Probenkammer wie in Figur 1 gezeigt kann beispielsweise so hergestellt werden, dass die Deckplatte einem Lösungsmitteldampf (als Quellmittel für das Polymer) ausgesetzt wird und anschließend mit der Bodenplatte, auf der sich beispielsweise die Kontaktelektroden, Mess- oder Anregungselektroden und Leiterbahnen befinden, insbesondere durch Verpressen verbunden wird.

Anstatt der oder zusätzlich zu den Leiterelementen der beispielhaften Probenkammer aus Figur 1, kann eine Probenkammer auch Halbleiterelemente umfassen. Insbesondere können Halbleiterelemente, beispielsweise ITO, als Heizelemente verwendet werden.

## Patentansprüche

1. Probenkammerhalter zum Haltern einer Probenkammer (101; 301; 401; 501) zur Untersuchung von Proben, wobei die Probenkammer (101; 301; 401; 501) umfasst:
eine Bodenplatte (107; 207) und eine damit verbundene Deckplatte (106; 206),
ein Probenreservoir (109) zur Aufnahme einer Flüssigkeit und/oder einer zu untersuchenden Probe, und
eine vollständig in der Bodenplatte (107; 207), in der Deckplatte (106; 206) oder zwischen Bodenplatte (107; 207) und Deckplatte (106; 206) angeordnete Kontaktelektrode (102; 202; 302; 402), die mit einem in und/oder auf der Probenkammer (101; 301; 401; 501) angeordneten Leiterelement (103; 203; 403) oder Halbleiterelement, stromleitend verbunden ist,
wobei die Bodenplatte (107; 207) und/oder die Deckplatte (106; 206) derart ausgebildet sind, dass von außen mit der Kontaktelektrode (102; 202; 302; 402) ein elektrischer Kontakt derart herstellbar ist, dass von außen durch die Bodenplatte (107; 207) oder durch die Deckplatte (106; 206) über die Kontaktelektrode (102; 202; 302; 402) eine stromleitende Verbindung mit dem Leiter- (103; 203; 403) oder Halbleiterelement herstellbar ist;
wobei die Probenkammer (101; 301; 401; 501) ein Loch aufweist, in dem die Kontaktelektrode (102; 202; 302; 402) angeordnet ist und wobei das Loch ein Sackloch (110; 310; 410) ist und die Kontaktelektrode (102; 202; 302; 402) am Boden und/oder an einer Seitenwand des Sacklochs (110; 310; 410) angeordnet ist;
wobei die Probenkammer (101; 301; 401; 501) eine Messelektrode (104; 204; 404) und/oder eine Anregungselektrode (105; 205) aufweist, welche jeweils mit einer Kontaktelektrode (102; 202; 302; 402) jeweils über ein Leiter- (103; 203; 403) oder Halbleiterelement stromleitend verbunden sind; und
wobei das Leiterelement (103; 203; 403) und/oder das Halbleiterelement vollständig zwischen der Bodenplatte (107; 207) und der Deckplatte (106; 206) oder vollständig auf der Außenseite der Probenkammer (101; 301; 401; 501) oder teilweise zwischen der Bodenplatte (107; 207) und der Deckplatte (106; 206) und teilweise auf der Außenseite der Probenkammer (101; 301; 401; 501) angeordnet sind; und
wobei der Probenkammerhalter umfasst:
ein erstes Rahmenelement (315; 415; 515) und ein zweites Rahmenelement (316; 416; 516),
ein elektrisches Kontaktelement (319; 419; 425; 519), welches am ersten (315; 415; 515) oder zweiten Rahmenelement (316; 416; 516) angeordnet ist,
eine Verbindungseinrichtung zum Verbinden des ersten und zweiten Rahmenelements, um einen zusammengesetzten Probenkammerhalter mit einer vorherbestimmten relativen Position der Rahmenelemente zueinander zu erhalten, wobei die Verbindungseinrichtung wenigstens ein magnetisches und/oder magnetisierbares Element (317; 318; 417; 418; 517) umfasst, und
wobei das erste (315; 415; 515) und das zweite Rahmenelement (316; 416; 516) derart ausgebildet sind, dass die Probenkammer (101; 301; 401; 501) im zusammengesetzten Probenkammerhalter zwischen erstem (315; 415; 515) und zweitem Rahmenelement (316; 416; 516) fixiert ist, und
wobei im zusammengesetzten Probenkammerhalter das elektrische Kontaktelement (319; 419; 425; 519) des Probenkammerhalters elektrisch leitend mit der Kontaktelektrode (102; 202; 302; 402) der fixierten Probenkammer (101; 301; 401; 501) verbunden ist.

2. Probenkammerhalter nach Anspruch 1, wobei das erste Rahmenelement (315; 415; 515) und/oder das zweite Rahmenelement (316; 416; 516) Vertiefungen (322; 422) zum Aufnehmen der Probenkammer (101; 301; 401; 501) aufweisen.

3. Probenkammerhalter nach Anspruch 1 oder 2, wobei das elektrische Kontaktelement (319; 419; 425; 519) des Probenkammerhalters einen Federkontakt umfasst.

4. Probenkammerhalter nach einem der Ansprüche 1 bis 3, wobei das erste Rahmenelement (315; 415; 515) und/oder das zweite Rahmenelement (316; 416; 516) derart ausgebildet sind, dass eine Probe in der Probenkammer (101; 301; 401; 501) mikroskopierbar ist.

5. Probenkammerhalter nach einem der Ansprüche 1 bis 4, wobei das erste Rahmenelement (315; 415; 515) und/oder das zweite Rahmenelement (316; 416; 516) ein Durchgangsloch (320; 321; 420; 421) oder einen transparenten Bereich, insbesondere aus Glas oder Kunststoff, im Bereich der Probenkammer (101; 301; 401; 501) umfasst.

6. System umfassend:
eine Probekammer (101; 301; 401; 501) umfassend:
eine Bodenplatte (107; 207) und eine damit verbundene Deckplatte (106; 206),
ein Probenreservoir (109) zur Aufnahme einer Flüssigkeit und/oder einer zu untersuchenden Probe, und
eine vollständig in der Bodenplatte (107; 207), in der Deckplatte (106; 206) oder zwischen Bodenplatte (107; 207) und Deckplatte (106; 206) angeordnete Kontaktelektrode (102; 202; 302; 402), die mit einem in und/oder auf der Probenkammer (101; 301; 401; 501) angeordneten Leiterelement (103; 203; 403) oder Halbleiterelement, stromleitend verbunden ist,
wobei die Bodenplatte (107; 207) und/oder die Deckplatte (106; 206) derart ausgebildet sind, dass von außen mit der Kontaktelektrode (102; 202; 302; 402) ein elektrischer Kontakt derart herstellbar ist, dass von außen durch die Bodenplatte (107; 207) oder durch die Deckplatte (106; 206) über die Kontaktelektrode (102; 202; 302; 402) eine stromleitende Verbindung mit dem Leiter- (103; 203; 403) oder Halbleiterelement herstellbar ist;
wobei die Probenkammer (101; 301; 401; 501) ein Loch aufweist, in dem die Kontaktelektrode (102; 202; 302; 402) angeordnet ist und wobei das Loch ein Sackloch (110; 310; 410) ist und die Kontaktelektrode (102; 202; 302; 402) am Boden und/oder an einer Seitenwand des Sacklochs (110; 310; 410) angeordnet ist;
wobei die Probenkammer (101; 301; 401; 501) eine Messelektrode (104; 204; 404) und/oder eine Anregungselektrode (105; 205) aufweist, welche jeweils mit einer Kontaktelektrode (102; 202; 302; 402) jeweils über ein Leiter- (103; 203; 403) oder Halbleiterelement stromleitend verbunden sind; und
wobei das Leiterelement (103; 203; 403) und/oder das Halbleiterelement vollständig zwischen der Bodenplatte (107; 207) und der Deckplatte (106; 206) oder vollständig auf der Außenseite der Probenkammer (101; 301; 401; 501) oder teilweise zwischen der Bodenplatte (107; 207) und der Deckplatte (106; 206) und teilweise auf der Außenseite der Probenkammer (101; 301; 401; 501) angeordnet sind; und
einen Probenkammernhalter nach einem der Ansprüche 1 bis 5.

## Claims

1. Sample chamber holder for holding a sample chamber (101; 301; 401; 501) for testing samples, the sample chamber (101; 301; 401; 501) comprising:
a bottom plate (107; 207) and a cover plate (106; 206) connected thereto,
a sample reservoir (109) for receiving a liquid and/or a sample to be tested, and
a contact electrode (102; 202; 302; 402) which is arranged entirely in the bottom plate (107; 207), in the cover plate (106; 206) or between the bottom plate (107; 207) and the cover plate (106; 206) and which is electrically conductively connected to a conductor element (103; 203; 403) or semiconductor element disposed in and/or on the sample chamber (101; 301; 401; 501),
wherein the bottom plate (107; 207) and/or the cover plate (106; 206) are designed in such a way that an electrical contact with the contact electrode (102; 202; 302; 402) can be established from the outside in such a way that an electrically conductive connection to the conductor element (103; 203; 403) or to the semiconductor element can be established from the outside through the bottom plate (107; 207) or through the cover plate (106; 206) via the contact electrode (102; 202; 302; 402);
wherein the sample chamber (101; 301; 401; 501) comprises a hole in which the contact electrode (102; 202; 302; 402) is arranged and wherein the hole is a blind hole (110; 310; 410) and the contact electrode (102; 202; 302; 402) is arranged on the bottom and/or on a side wall of the blind hole (110; 310; 410);
wherein the sample chamber (101; 301; 401; 501) comprises a measuring electrode (104; 204; 404) and/or an excitation electrode (105; 205) each of which are electrically conductively connected to a contact electrode (102; 202; 302; 402) by a conductor element (103; 203; 403) or semiconductor element; and
wherein the conductor element (103; 203; 403) and/or the semiconductor element are disposed entirely between the bottom plate (107; 207) and the cover plate (106; 206) or entirely on the outer side of the sample chamber (101; 301; 401; 501) or partially between the bottom plate (107; 207) and the cover plate (106; 206) and partially on the outer side of the sample chamber (101; 301; 401; 501); and
wherein the sample chamber holder comprises:
a first frame element (315; 415; 515) and a second frame element (316; 416; 516),
an electrical contact element (319; 419; 425; 519) which is disposed on the first (315; 415; 515) or second frame element (316; 416; 516),
a connecting device for connecting the first and second frame element so as to obtain an assembled sample chamber holder with a predetermined relative position of the frame elements with respect to each other, wherein the connecting device comprises at least one magnetic and/or magnetizable element (317; 318; 417; 418; 517), and
wherein the first (315; 415; 515) and the second frame element (316; 416; 516) are designed in such a way that the sample chamber (101; 301; 401; 501) is fixed in the assembled sample chamber holder between the first (315; 415; 515) and the second frame element (316; 416; 516), and
wherein, in the assembled sample chamber holder, the electrical contact element (319; 419; 425; 519) of the sample chamber holder is electrically conductively connected to the contact electrode (102; 202; 302; 402) of the fixed sample chamber (101; 301; 401; 501).

2. Sample chamber holder according to claim 1, wherein the first frame element (315; 415; 515) and/or the second frame element (316; 416; 516) include recesses (322; 422) for receiving the sample chamber (101; 301; 401; 501).

3. Sample chamber holder according to claim 1 or 2, wherein the electrical contact element (319; 419; 425; 519) of the sample chamber holder comprises a spring contact.

4. Sample chamber holder according to one of claims 1 to 3, wherein the first frame element (315; 415; 515) and/or the second frame element (316; 416; 516) are adapted to allow a microscopical examination of a sample in the sample chamber (101; 301; 401; 501).

5. Sample chamber holder according to one of claims 1 to 4, wherein the first frame element (315; 415; 515) and/or the second frame element (316; 416; 516) include a through-hole (320; 321; 420; 421) or a transparent area, specifically made of glass or plastic, in the area of the sample chamber (101; 301; 401; 501).

6. System comprising:
a sample chamber (101; 301; 401; 501) comprising:
a bottom plate (107; 207) and a cover plate (106; 206) connected thereto,
a sample reservoir (109) for receiving a liquid and/or a sample to be tested, and
a contact electrode (102; 202; 302; 402) which is arranged entirely in the bottom plate (107; 207), in the cover plate (106; 206) or between the bottom plate (107; 207) and the cover plate (106; 206) and which is electrically conductively connected to a conductor element (103; 203; 403) or semiconductor element disposed in and/or on the sample chamber (101; 301; 401; 501),
wherein the bottom plate (107; 207) and/or the cover plate (106; 206) are designed in such a way that an electrical contact with the contact electrode (102; 202; 302; 402) can be established from the outside in such a way that an electrically conductive connection to the conductor element (103; 203; 403) or to the semiconductor element can be established from the outside through the bottom plate (107; 207) or through the cover plate (106; 206) via the contact electrode (102; 202; 302; 402);
wherein the sample chamber (101; 301; 401; 501) comprises a hole in which the contact electrode (102; 202; 302; 402) is arranged and wherein the hole is a blind hole (110; 310; 410) and the contact electrode (102; 202; 302; 402) is arranged on the bottom and/or on a side wall of the blind hole (110; 310; 410);
wherein the sample chamber (101; 301; 401; 501) comprises a measuring electrode (104; 204; 404) and/or an excitation electrode (105; 205) each of which are electrically conductively connected to a contact electrode (102; 202; 302; 402) by a conductor element (103; 203; 403) or semiconductor element; and
wherein the conductor element (103; 203; 403) and/or the semiconductor element are disposed entirely between the bottom plate (107; 207) and the cover plate (106; 206) or entirely on the outer side of the sample chamber (101; 301; 401; 501) or partially between the bottom plate (107; 207) and the cover plate (106; 206) and partially on the outer side of the sample chamber (101; 301; 401; 501); and
a sample chamber holder according to one of claims 1 to 5.

## Revendications

1. Support de chambre d'échantillon pour supporter une chambre d'échantillon (101 ; 301 ; 401 ; 501) destinée à l'analyse d'échantillons, dans lequel la chambre d'échantillon (101 ; 301 ; 401 ; 501) comprend :
une plaque de base (107 ; 207) et une plaque de recouvrement (106 ; 206) qui lui est reliée,
un réservoir d'échantillon (109) pour recevoir un fluide et/ou un échantillon à analyser, et
une électrode de contact (102 ; 202 ; 302 ; 402) complètement placée dans la plaque de base (107 ; 207), dans la plaque de recouvrement (106 ; 206) ou entre la plaque de base (107 ; 207) et la plaque de recouvrement (106 ; 206), qui est connectée électriquement à un élément conducteur (103 ; 203 ; 403) ou semi-conducteur agencé dans et/ou sur la chambre d'échantillon (101 ; 301 ; 401 ; 501),
dans lequel la plaque de base (107 ; 207) et/ou la plaque de recouvrement (106 ; 206) sont constituées de telle sorte qu'un contact électrique peut être établi depuis l'extérieur avec l'électrode de contact (102 ; 202 ; 302 ; 402) de façon à ce qu'une connexion électrique avec l'élément conducteur (103 ; 203 ; 403) ou semi-conducteur puisse être établie depuis l'extérieur à travers la plaque de base (107 ; 207) ou à travers la plaque de recouvrement (106 ; 206) via l'électrode de contact (102 ; 202 ; 302 ; 402) ;
dans lequel la chambre d'échantillon (101 ; 301 ; 401 ; 501) comporte un trou où est agencée l'électrode de contact (102 ; 202 ; 302 ; 402) et dans lequel le trou est un trou borgne (110 ; 310 ; 410) et l'électrode de contact (102 ; 202 ; 302 ; 402) est agencée au fond et/ou sur une paroi latérale du trou borgne (110 ; 310 ; 410) ;
dans lequel la chambre d'échantillon (101 ; 301 ; 401 ; 501) comporte une électrode de mesure (104 ; 204 ; 404) et/ou une électrode d'excitation (105 ; 205) qui sont chacune connectées électriquement à une électrode de contact (102 ; 202 ; 302 ; 402) via un élément conducteur (103 ; 203 ; 403) ou semi-conducteur correspondant ; et
dans lequel l'élément conducteur (103 ; 203 ; 403) et/ou l'élément semi-conducteur sont agencés complètement entre la plaque de base (107 ; 207) et la plaque de recouvrement (106 ; 206), ou complètement sur le côté externe de la chambre d'échantillon (101 ; 301 ; 401 ; 501), ou partiellement entre la plaque de base (107 ; 207) et la plaque de recouvrement (106 ; 206), et partiellement sur le côté externe de la chambre d'échantillon (101 ; 301 ; 401 ; 501) ; et
dans lequel le support de chambre d'échantillon comprend :
un premier élément de châssis (315 ; 415 ; 515) et un deuxième élément de châssis (316 ; 416 ; 516),
un élément de contact électrique (319 ; 419 ; 425 ; 519) qui est agencé sur le premier élément de châssis (315 ; 415 ; 515) ou le deuxième élément de châssis (316 ; 416 ; 516),
un dispositif de connexion pour connecter les premier et deuxième éléments de châssis pour obtenir un support de chambre d'échantillon assemblé avec une position relative prédéterminée des éléments de châssis entre eux, dans lequel le dispositif de connexion comprend au moins un élément magnétique et/ou magnétisable (317 ; 318 ; 417 ; 418 ; 517)), et
dans lequel les premier (315 ; 415 ; 515) et deuxième éléments de châssis (316 ; 416 ; 516) sont construits de telle façon que la chambre d'échantillon (101 ; 301 ; 401 ; 501) est fixée dans le support de chambre d'échantillon assemblé entre les premier (315 ; 415 ; 515) et deuxième éléments de châssis (316 ; 416 ; 516), et
dans lequel, dans le support de chambre d'échantillon assemblé, l'élément de contact électrique (319 ; 419 ; 425 ; 519) du support de chambre d'échantillon est connecté électriquement à l'électrode de contact (102 ; 202 ; 302 ; 402) de la chambre d'échantillon fixée (101 ; 301 ; 401 ; 501).

2. Support de chambre d'échantillon selon la revendication 1, dans lequel le premier élément de châssis (315 ; 415 ; 515) et/ou le deuxième élément de châssis (316 ; 416 ; 516) comportent des renfoncements (322 ; 422) pour loger la chambre d'échantillon (101 ; 301 ; 401 ; 501).

3. Support de chambre d'échantillon selon la revendication 1 ou 2, dans lequel l'élément de contact électrique (319 ; 419 ; 425 ; 519) du support de chambre d'échantillon comporte un contact à ressort.

4. Support de chambre d'échantillon selon l'une des revendications 1 à 3, dans lequel le premier élément de châssis (315 ; 415 ; 515) et/ou le deuxième élément de châssis (316 ; 416 ; 516) sont constitués de telle sorte qu'un échantillon dans la chambre d'échantillon (101 ; 301 ; 401 ; 501) peut être observé au microscope.

5. Support de chambre d'échantillon selon l'une des revendications 1 à 4, dans lequel le premier élément de châssis (315 ; 415 ; 515) et/ou le deuxième élément de châssis (316 ; 416 ; 516) comportent un trou traversant (320 ; 321 ; 420 ; 421) ou une zone transparente, en particulier en verre ou en plastique, dans la zone de la chambre d'échantillon (101 ; 301 ; 401 ; 501).

6. Système comprenant :
une chambre d'échantillon (101 ; 301 ; 401 ; 501) comprenant :
une plaque de base (107 ; 207) et une plaque de recouvrement (106 ; 206) qui lui est reliée,
un réservoir d'échantillon (109) pour recevoir un fluide et/ou un échantillon à analyser, et
une électrode de contact (102 ; 202 ; 302 ; 402) complètement placée dans la plaque de base (107 ; 207), dans la plaque de recouvrement (106 ; 206) ou entre la plaque de base (107 ; 207) et la plaque de recouvrement (106 ; 206), qui est connectée électriquement à un élément conducteur (103 ; 203 ; 403) ou semi-conducteur agencé dans et/ou sur la chambre d'échantillon (101 ; 301 ; 401 ; 501),
dans lequel la plaque de base (107 ; 207) et/ou la plaque de recouvrement (106 ; 206) sont constituées de telle sorte qu'un contact électrique peut être établi depuis l'extérieur avec l'électrode de contact (102 ; 202 ; 302 ; 402) de façon à ce qu'une connexion électrique avec l'élément conducteur (103 ; 203 ; 403) ou semi-conducteur puisse être établie depuis l'extérieur à travers la plaque de base (107 ; 207) ou à travers la plaque de recouvrement (106 ; 206) via l'électrode de contact (102 ; 202 ; 302 ; 402) ;
dans lequel la chambre d'échantillon (101 ; 301 ; 401 ; 501) comporte un trou où est agencée l'électrode de contact (102 ; 202 ; 302 ; 402) et dans lequel le trou est un trou borgne (110 ; 310 ; 410) et l'électrode de contact (102 ; 202 ; 302 ; 402) est agencée au fond et/ou sur une paroi latérale du trou borgne (110 ; 310 ; 410) ;
dans lequel la chambre d'échantillon (101 ; 301 ; 401 ; 501) comporte une électrode de mesure (104 ; 204 ; 404) et/ou une électrode d'excitation (105 ; 205) qui sont chacune connectées électriquement à une électrode de contact (102 ; 202 ; 302 ; 402) via un élément conducteur (103 ; 203 ; 403) ou semi-conducteur correspondant ; et
dans lequel l'élément conducteur (103 ; 203 ; 403) et/ou l'élément semi-conducteur sont agencés complètement entre la plaque de base (107 ; 207) et la plaque de recouvrement (106 ; 206), ou complètement sur le côté externe de la chambre d'échantillon (101 ; 301 ; 401 ; 501), ou partiellement entre la plaque de base (107 ; 207) et la plaque de recouvrement (106 ; 206), et partiellement sur le côté externe de la chambre d'échantillon (101 ; 301 ; 401 ; 501) ; et
un support de chambre d'échantillon selon l'une des revendications 1 à 5.
